# EUROPEAN PATENT APPLICATION

(11) **EP 2 712 577 A1**
(43) Date of publication of application: **02.04.2014**
(21) Application number: 13186533.9
(22) Date of filing: 27.09.2013
(51) Int. Cl.: A61F 2/00

(54) **Porous substrate with ferromagnetic darts**

(30) Priority: 28.09.2012 US 201261706888 P; 11.09.2013 US 201314023501
(71) Applicant: Covidien LP, Mansfield, MA 02048 (US)
(72) Inventor: Sargeant, Timothy, Guildford, CT Connecticut 06437 (US); Thomas, Jonathan, New Haven, CT Connecticut 06511 (US)
(74) Representative: Maschio, Antonio

(57) **Abstract**

A surgical implant includes a porous substrate including a first surface and a second surface, and a plurality of ferromagnetic darts (16) extending from at least one of the first and second surfaces. The surgical implant may be used in combination with an electromagnet that is configured to attract the plurality of darts for fixing the surgical implant to tissue.

## Description

### CROSS-REFERENCE TO RELATED APPLICATIONS

This application claims the benefit of and priority to U.S. Provisional Patent Application No. 61/706,888, filed September 28, 2012, the entire disclosure of which is incorporated by reference herein.

### TECHNICAL FIELD

The present disclosure relates to medical devices with ferromagnetic properties. More particularly, the present disclosure relates to surgical implants including a porous substrate having ferromagnetic darts extending therefrom, and systems and methods of using the same for tissue fixation.

### BACKGROUND

Techniques for repairing damaged or diseased tissue are widespread in medicine. Wound closure devices, such as sutures and staples, as well as other repair devices like mesh or patch reinforcements, are frequently used for repair. For example, in the case of hernias, a surgical mesh or patch is commonly used to reinforce the abdominal wall. The surgical mesh is generally sized to extend across the defect and is adapted to flex or bend in order to conform to the abdominal wall.

There are great concerns over ease of deployment, positioning, and placement of hernia meshes. Surgeons often struggle with the placement and fixation of a surgical mesh in laparoscopic procedures because the mesh will not readily adhere or fix to the abdominal wall. Surgical meshes are typically fixed to surrounding tissue with tissue fixation devices (e.g., sutures, staples, tacks), however, difficulties and problems may arise with the use of these devices.

For example, sutures can often cause excessive post-operative pain. Sutures have relatively low compliance compared to abdominal tissues, and therefore sutures may "pinch" when muscle tissue contracts. Staples and tacks may occasionally dislodge from tissue thereby irritating tissue as they move within the body, and may result in re-herniation, dislodging, or repositioning of the surgical mesh relative to the tissue and/or viscera entering the defect.

It would be advantageous to provide a surgical implant having integrated tissue fixation elements that can be deployed, positioned, and placed at a desired tissue site without the need for external tissue fixation devices.

### SUMMARY

A surgical implant, system, and method of using the same are disclosed. The surgical implant of the present disclosure includes a porous substrate including a first surface and a second surface, and a plurality of ferromagnetic darts extending from at least one of the first and second surfaces. The surgical implant may be used in combination with an electromagnet that is configured to attract the plurality of darts for fixing the surgical implant to tissue.

In a method of use, the surgical implant may be placed adjacent tissue such that the darts face the tissue. The electromagnet may be placed on an opposite side of the tissue. Activation of the electromagnet attracts the plurality of darts towards the electromagnet and into the tissue.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiments given below, serve to explain the principles of the disclosure:

FIG. 1 is a cross-sectional schematic illustration of a surgical implant in accordance with the present disclosure;

FIG. 2 is a perspective view of a surgical implant in accordance with an embodiment of the present disclosure;

FIG. 3 is a perspective view of a surgical implant in accordance with another embodiment of the present disclosure;

FIG. 4 is a cross-sectional view of a surgical implant in accordance with yet another embodiment of the present disclosure; and

FIGS. 5A through 5B are schematic illustrations of an exemplary system and method of fixing a surgical implant at a desired location against tissue.

### DETAILED DESCRIPTION OF THE EMBODIMENTS

Embodiments of the surgical implant, and a system and method of using the same will now be described in detail with reference to the drawings wherein like reference numerals identify similar or like elements throughout the several view. While the present discussion and figures below depict the surgical implant as a hernia mesh, the presently disclosed surgical implant may be any medical device, such as scaffolds, grafts, patches, slings, pledgets, growth matrices, drug delivery devices, wound plugs, and, in general, soft tissue repair devices and surgical prosthesis that can be used in medical/surgical procedures.

Surgical implants in accordance with the present disclosure include a porous substrate and ferromagnetic darts extending therefrom that aid in implant placement and fixation in tissue.

Porous substrates in accordance with the present disclosure may be a mesh, fibrous sheet, patch, foam, film, or composite thereof. The term "porous" as used herein may define openings and spacings which are present as a surface characteristic or a bulk material property, partially or completely penetrating the substrate. Suitable materials for forming a porous substrate include, but are not limited to fibrous structures (e.g., knitted structures, woven structures, non-woven structures, etc.), foams (e.g., open or closed cell foams), and perforated films. Use of a porous substrate may allow for quicker healing through the openings formed therein.

The porous substrate should have the following characteristics: sufficient tensile strength to support a fascial wall during repair of a defect in the fascial wall causing a hernia; sufficiently inert to avoid foreign body reactions when retained in the body for long periods of time; easily sterilized to prevent the introduction of infection when the substrate is implanted in the body; and suitably easy handling characteristics for placement in the desired location in the body. The porous substrate should be sufficiently pliable to conform to a fascial wall and flex with movement of the wall, while being sufficiently rigid to retain its shape. The porous substrate should also be sufficiently strong to avoid tearing of portions thereof.

In embodiments, the porous substrate is fabricated from a textile including yarns. Yarns forming the porous substrate may be monofilament or multifilament yarns which may be made of any suitable biocompatible material. In some embodiments, the yarns include at least two filaments which may be arranged to create openings therebetween, the yarns also being arranged relative to each other to form openings in the porous substrate. Alternatively, the porous substrate may be formed from a continuous yarn that is arranged in loops that give rise to the openings in the porous substrate. The use of a porous substrate having yarns spaced apart in accordance with the present disclosure has the advantage of reducing the foreign body mass that is implanted in the body, while maintaining sufficient tensile strength to securely support the defect and tissue being repaired by the porous substrate. Moreover, the openings of the porous substrate of the present disclosure may be sized to permit fibroblast through-growth and ordered collagen laydown, resulting in integration of the porous substrate into the body. Thus, the spacing between the yarns may vary depending on the surgical application and desired implant characteristics as envisioned by those skilled in the art. Moreover, due to the variety of sizes of defects, and of the various fascia that may need repair, the porous substrate may be of any suitable size.

The yarns may be braided, twisted, aligned, fused, or otherwise joined to form a variety of different porous substrate shapes. In embodiments in which at least two filaments form a yarn, the filaments may be drawn, oriented, crinkled, twisted, braided, commingled or air entangled to form the yarn. The yarns may be woven, knitted, interlaced, braided, or formed into a porous substrate by non-woven techniques. The structure of the porous substrate will vary depending upon the assembling technique utilized to form the porous substrate, as well as other factors such as the type of fibers used, the tension at which the yarns are held, and the mechanical properties required of the porous substrate.

In embodiments, knitting may be utilized to form a porous substrate of the present disclosure. Knitting involves, in embodiments, the intermeshing of yarns to form loops or inter-looping of the yarns. In some embodiments, yarns may be warp-knitted thereby creating vertical interlocking loop chains and/or may be weft-knitted thereby creating rows of interlocking loop stitches across the porous substrate. In other embodiments, weaving may be utilized to form a porous substrate of the present disclosure. Weaving may include, in embodiments, the intersection of two sets of straight yarns, warp and weft, which cross and interweave at right angles to each other, or the interlacing of two yarns at right angles to each other. In some embodiments, the yarns may be arranged to form a net porous substrate which has isotropic or near isotropic tensile strength and elasticity.

In embodiments, the yarns may be nonwoven and formed by mechanically, chemically, or thermally bonding the yarns into a sheet or web in a random or systematic arrangement. For example, yarns may be mechanically bound by entangling the yarns to form the porous substrate by means other than knitting or weaving, such as matting, pressing, stitch-bonding, needlepunching, or otherwise interlocking the yarns to form a binderless network. In other embodiments, the yarns of the porous substrate may be chemically bound by use of an adhesive, such as a hot melt adhesive, or thermally bound by applying a binder, such as a powder, paste, or melt, and melting the binder on the sheet or web of yarns.

The porous substrate may be fabricated from any biodegradable and/or non-biodegradable polymer that can be used in surgical procedures. The term "biodegradable" as used herein is defined to include both bioabsorbable and bioresorbable materials. By biodegradable, it is meant that the material decomposes, or loses structural integrity under body conditions (e.g., enzymatic degradation or hydrolysis) or is broken down (physically or chemically) under physiologic conditions in the body such that the degradation products are excretable or absorbable by the body. Absorbable materials are absorbed by biological tissues and disappear in vivo at the end of a given period, which can vary for example from hours to several months, depending on the chemical nature of the material. It should be understood that such materials include natural, synthetic, bioabsorbable, and/or certain non-absorbable materials, as well as combinations thereof.

Representative natural biodegradable polymers include: polysaccharides such as alginate, dextran, chitin, chitosan, hyaluronic acid, cellulose, collagen, gelatin, fucans, glycosaminoglycans, and chemical derivatives thereof (substitutions and/or additions of chemical groups include, for example, alkyl, alkylene, amine, sulfate, hydroxylations, carboxylations, oxidations, and other modifications routinely made by those skilled in the art); catgut; silk; linen; cotton; and proteins such as albumin, casein, zein, silk, soybean protein, and copolymers and blends thereof; alone or in combination with synthetic polymers.

Synthetically modified natural polymers which may be used to form the yarns include cellulose derivatives such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, and cellulose sulfate sodium salt.

Representative synthetic biodegradable polymers which may be utilized to form yarns include polyhydroxy acids prepared from lactone monomers such as glycolide, lactide, caprolactone, ε-caprolactone, valerolactone, and δ-valerolactone, carbonates (e.g., trimethylene carbonate, tetramethylene carbonate, and the like), dioxanones (e.g., 1,4-dioxanone and p-dioxanone), 1,dioxepanones (e.g., 1,4-dioxepan-2-one and 1,5-dioxepan-2-one), and combinations thereof. Polymers formed therefrom include: polylactides; poly(lactic acid); polyglycolides; poly(glycolic acid); poly(trimethylene carbonate); poly(dioxanone); poly(hydroxybutyric acid); poly(hydroxyvaleric acid); poly(lactide-co-(ε-caprolactone-)); poly(glycolide-co-(ε-caprolactone)); polycarbonates; poly(pseudo amino acids); poly(amino acids); poly(hydroxyalkanoate)s such as polyhydroxybutyrate, polyhydroxyvalerate, poly(3-hydroxybutyrate-co-3-hydroxyvalerate), polyhydroxyoctanoate, and polyhydroxyhexanoate; polyalkylene oxalates; polyoxaesters; polyanhydrides; polyester anyhydrides; polyortho esters; and copolymers, block copolymers, homopolymers, blends, and combinations thereof.

Some non-limiting examples of suitable non-degradable materials from which the porous substrate may be made include polyolefins such as polyethylene (including ultra high molecular weight polyethylene) and polypropylene including atactic, isotactic, syndiotactic, and blends thereof; polyethylene glycols; polyethylene oxides; polyisobutylene and ethylene-alpha olefin copolymers; fluorinated polyolefins such as fluoroethylenes, fluoropropylenes, fluoroPEGSs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polybutester; polytetramethylene ether glycol; 1,4-butanediol; polyurethanes; acrylic polymers; methacrylics; vinyl halide polymers such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; etheylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; and copolymers and combinations thereof.

A plurality of ferromagnetic darts extends from the porous substrate. The darts include a base portion that is fixedly associated with a surface of the porous substrate, a body portion extending therefrom, and a head portion that is configured for tissue fixation. The head portion may include a maximum width that is larger than the width of the body portion of the dart to aid in tissue fixation, and may taper into a sharp or spiked tip to facilitate insertion into tissue. The darts may be coupled to the porous substrate by conventional means, such as for example, ultrasonic welding, lamination, adhesive bonding, or by monolithically forming the darts with the porous substrate. The darts may be porous or non-porous, and are partially or fully formed from a metal having ferromagnetic properties.

In embodiments, a dart may be completely formed from an absorbable and/or non-absorbable ferromagnetic metal. Suitable metals include iron ore (magnetite or lodestone), cobalt and nickel, rare earth metals like gadolinium and dysprosium, and alloys thereof. The darts may also be made from composite materials such as ceramic or ferrite, alnico (a combination of aluminum, nickel and cobalt with iron), or triconal (a combination of titanium, cobalt, nickel and aluminum with iron).

In embodiments, a dart may be formed from a polymeric material including ferromagnetic metal particles. The ferromagnetic particles may be in the nanometer to micrometer-size range. The polymer may be any biodegradable and/or non-biodegradable polymer as described above, and may be the same or different from the polymer forming the porous substrate. In embodiments, ferromagnetic metal particles may be freely admixed or coextruded with the polymer forming the dart, or may be tethered to the polymer through any suitable chemical bond. In some embodiments, the ferromagnetic metal particles may be spray or dip coated on a formed polymeric dart.

The porous substrate and/or darts may be used to deliver therapeutic agents into the tissue. In general, therapeutic agents may be incorporated into the porous substrate and/or darts during manufacture or formation of the porous substrate and/or dart, such as by free solution, suspension, liposomal delivery, microspheres, etc., or by coating a surface of the porous substrate and/or dart, or selective regions thereof, such as by polymer coating, dry coating, freeze drying, or applying the coating directly to the porous substrate and/or dart surface. In embodiments, at least one therapeutic agent may be combined with a component of an absorbable porous substrate and/or dart to provide release of the therapeutic agent via degradation of the surgical implant. In other embodiments, therapeutic agents may be coated on an absorbable or non-absorbable component of the porous substrate and/or dart.

Therapeutic agents include any substance or mixture of substances that have clinical use. Consequently, therapeutic agents may or may not have pharmacological activity per se, e.g., a dye. Alternatively, a therapeutic agent could be any agent which provides a therapeutic or prophylactic effect; a compound that affects or participates in tissue growth, cell growth and/or cell differentiation; a compound that may be able to invoke or prevent a biological action such as an immune response; or a compound that could play any other role in one or more biological processes. A variety of therapeutic agents may be incorporated into the surgical implant of the present disclosure. Moreover, any agent which may enhance tissue repair, limit the risk of sepsis, and modulate the mechanical properties of the surgical implant (e.g., the swelling rate in water, tensile strength, etc.) may be added during the preparation of the surgical implant or may be coated thereon.

Examples of classes of therapeutic agents which may be utilized in accordance with the present disclosure include antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, and enzymes. It is also intended that combinations of therapeutic agents may be used.

Other therapeutic agents which may be in the present disclosure include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists such as naltrexone and naloxone; anti-cancer agents; anticonvulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable therapeutic agents which may be included in the present disclosure include: viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (IL-2, IL-3, IL-4, IL-6); interferons (β-IFN, (α-IFN and γ-IFN)); erythropoietin; nucleases; tumor necrosis factor; colony stimulating factors (e.g., GCSF, GM-CSF, MCSF); insulin; anti-tumor agents and tumor suppressors; blood proteins; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); protein inhibitors; protein antagonists; protein agonists; nucleic acids such as antisense molecules, DNA, and RNA; oligonucleotides; and ribozymes.

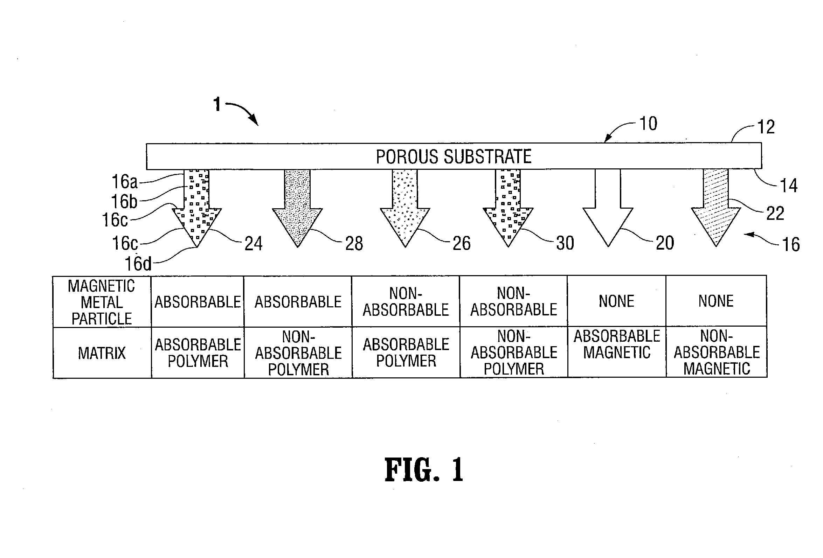

Turning now to FIG. 1, a surgical implant 1 in accordance with an embodiment of the present disclosure is illustrated. The surgical implant 1 includes a porous substrate 10 having a first surface 12 and a second surface 14, opposite the first surface 12. The porous substrate 10 is shown as substantially rectangular, however, the porous substrate 10 can be any dimension and shape for the envisaged application of use. A plurality of darts 16 extend from the second surface 14 of the porous substrate 10. While the darts are shown as extending substantially vertically from the second surface 14 of the porous substrate 10, it should be understood that the darts 16 may extend at any angle relative to the porous substrate 10 for grasping tissue, and may be provided on any tissue contacting portion of the porous substrate 10.

Each dart 16 includes a base portion 16a coupled to the porous substrate 10, a body portion 16b extending from the base portion 16a, and a head portion 16c extending from the body portion 16b. The head portion 16c is illustrated as an arrowhead having a substantially triangular shape, and includes a tapered tip 16d for penetrating tissue and a proximal end 16e having a maximum diameter that is larger than the diameter of the body portion 16b for fixing the dart 16 to tissue. Additional shapes are contemplated for the darts such as spiked naps or hooks.

As shown in FIG. 1, many different material combinations for the darts 16 are contemplated. The darts 16 may be made from an absorbable ferromagnetic metal 20 or a non-absorbable ferromagnetic metal 22. The darts 16 may also be made from an absorbable polymer having absorbable 24 or non-absorbable 26 magnetic particles. Alternatively, darts 16 may be made from a non-absorbable polymer having absorbable 28 or non-absorbable 30 ferromagnetic particles. As shown in FIG. 1, darts 16 including different material combinations may all be incorporated into one surgical implant 1. Alternatively, based on the surgical procedure, the darts of a surgical implant may all be formed from identical materials.

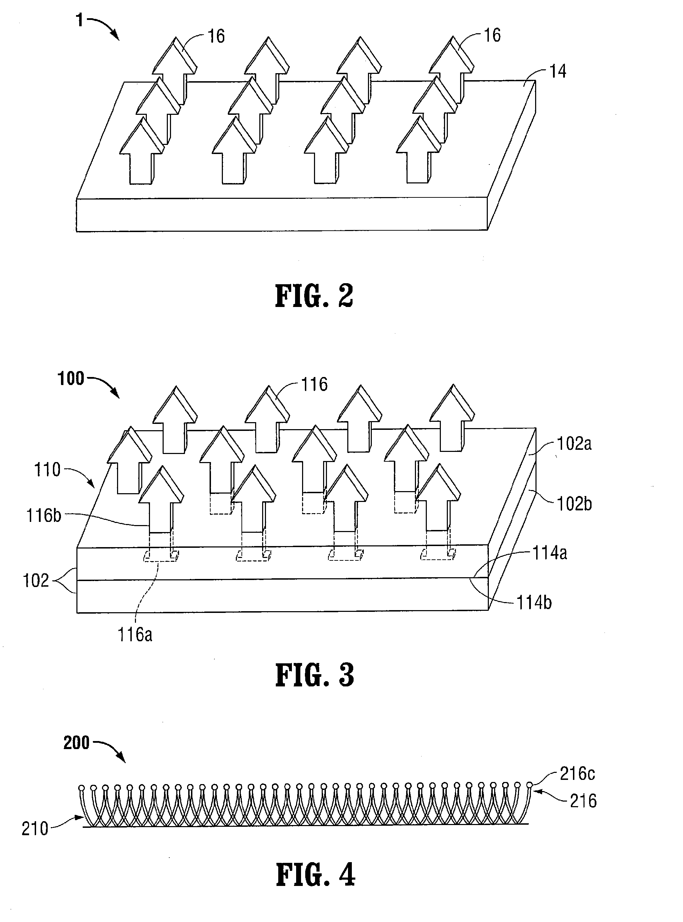

As shown in FIG. 2, the configuration of the darts 16 extending from the second surface 14 of the porous substrate 10 of a surgical instrument 1 may vary. The darts 16 may be positioned in a linear configuration horizontally and vertically. Alternatively, darts may be positioned diagonally along the second surface 14. In further embodiments, darts may be positioned along an outer perimeter of a porous substrate to grasp tissue surrounding a tissue defect.

FIG. 3 shows an embodiment of a surgical implant 100 having a layered porous substrate 110. Porous substrate includes two layers 102, a top layer 102a and a bottom layer 102b. The layers 102 are stacked vertically to provide additional support to the tissue during the healing process. Each of the top and bottom layers 102a, 102b have an inner surface 114a and an outer surface 114b such that the inner surface 114a of the top layer 102a juxtaposes the inner surface 114b of the bottom layer 102b. In this embodiment, the darts 116 include a base portion 116a having a diameter that is larger than the diameter of the body portion 116b so that the darts 116 may be mechanically coupled between the inner surface 114b of the bottom layer 102b and the inner surface 114a of the top layer 102a of the surgical implant 100.

FIG. 4 shows an embodiment of a surgical implant 200 having darts 216 that are integrally formed with a porous substrate 210. Porous substrate 210 may be a self-fixating substrate formed from a knit having darts 216 formed on one surface of the porous substrate 210 that protrude perpendicularly with respect to the porous substrate 210. An example of a self-fixation mesh which may be utilized as the porous substrate of the surgical implant of the present disclosure is Parietex Progrip^{™} Self-fixating Mesh, commercially available from Covidien. The darts 216 each have a substantially rectilinear body portion and, at the free end of this body, a head portion 216c of greater width than that of the body portion, that mechanical grip or hook tissue. The head portions 216c may be made from and/or coated with ferromagnetic metal materials and/or particles in a manner discussed above.

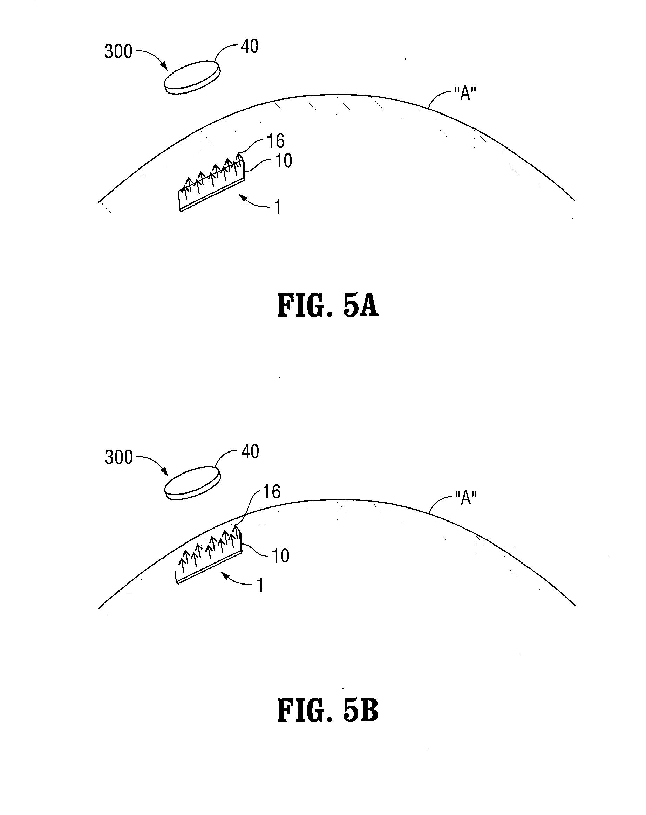

FIGS. 5A and 5B show an exemplary system and method for fixing a surgical implant of the present disclosure to tissue. System 300 includes a surgical implant, such as surgical implant 1 (FIG. 2) described above, and an external electromagnet 40 configured to attract the ferromagnetic darts 16 of the surgical implant 1. Electromagnet 40 and darts 16 have different polarities in order attract each other causing a magnetic force to pull the darts 16 towards the electromagnet 40.

As illustrated in FIG. 5A, surgical implant 1 is positioned internally within the abdominal cavity near the abdominal wall "A" requiring repair. An external electromagnet 40 is placed externally of the desired location of the surgical implant 1, on an opposing side of the abdominal tissue "A" and the darts 16. Electromagnet 40 is turned on or activated to attract the ferromagnetic particles of the darts 16. The darts 16 penetrate the tissue of the abdominal wall "A" thereby adhering the porous substrate 10 thereto, as illustrated in FIG. 5B.

If needed, the surgeon may reposition the surgical implant 1, by deactivating the electromagnet 40 and mechanically pulling the porous substrate 10 away from the abdominal wall "A." The surgeon may then reposition the surgical implant 1 and activate the electromagnet 40 to again penetrate the tissue of the abdominal wall "A" and adhere the porous substrate 10 thereto.

While several embodiments of the disclosure have been described, it is not intended that the disclosure be limited thereto, as it is intended that the disclosure be as broad in scope as the art will allow and that the specification be read likewise. Therefore, the above description should not be construed as limiting, but merely as exemplifications of embodiments of the present disclosure. Various modifications and variations of the surgical implant, and system and method of use, will be apparent to those skilled in the art from the foregoing detailed description. Such modifications and variations are intended to come within the scope and spirit of the claims appended hereto.

The invention may be described by reference to the following numbered paragraphs: -
1. A surgical implant comprising:
   a porous substrate including a first surface and a second surface, opposite the first surface; and
   a plurality of ferromagnetic darts extending from at least one of the first and second surfaces.
2. The surgical implant of paragraph 1, wherein at least one of the plurality of ferromagnetic darts is made from a bioabsorbable material.
3. The surgical implant of paragraph 2, wherein the at least one dart is formed from a ferromagnetic metal.
4. The surgical implant of paragraph 3, wherein the metal is iron or alloys thereof.
5. The surgical implant of paragraph 3, wherein the metal is selected from the group consisting of cobalt, nickel, gadolinium, dysprosium, and alloys thereof.
6. The surgical implant of paragraph 2, wherein the bioabsorbable material is a polymer including ferromagnetic particles.
7. The surgical implant of paragraph 6, wherein the ferromagnetic particles are admixed with the polymer.
8. The surgical implant of paragraph 6, wherein the ferromagnetic particles are coated on the polymer.
9. The surgical implant of paragraph 1, wherein at least one of the plurality of darts is made from a non-absorbable material.
10. The surgical implant of paragraph 9, wherein the at least one dart is formed from a ferromagnetic metal.
11. The surgical implant of paragraph 10, wherein the metal is iron or alloys thereof.
12. The surgical implant of paragraph 10, wherein the metal is selected from the group consisting of cobalt, nickel, gadolinium, dysprosium, and alloys thereof.
13. The surgical implant of paragraph 9, wherein the non-absorbable material is a polymer including ferromagnetic particles.
14. The surgical implant of paragraph 13, wherein the ferromagnetic particles are admixed with the polymer.
15. The surgical implant of paragraph 13, wherein the ferromagnetic particles are coated on the polymer.
16. The surgical implant of paragraph 1, wherein the darts each include:
   a base portion fixedly associated with the porous substrate;
   a body portion extending from the base portion, and
   a head portion extending from the body portion.
17. The surgical implant of paragraph 16, wherein the head portion includes a tapered tip.
18. The surgical implant of paragraph 17, wherein the head portion of the dart includes a proximal end having a diameter that is larger than the diameter of the body portion.
19. A system for securing a surgical implant to tissue, the system comprising:
   a surgical implant including a porous substrate having a plurality of ferromagnetic darts extending therefrom; and
   an electromagnet configured to attract the plurality of darts.
20. A method for securing a surgical implant to tissue, the method comprising:
   providing a surgical implant including a porous substrate having a plurality of ferromagnetic darts extending therefrom;
   placing the surgical implant adjacent tissue such that the darts face the tissue;
   placing an electromagnet on an opposite side of the tissue; and
   activating the electromagnet, thereby attracting the plurality of darts towards the electromagnet and into the tissue.

## Claims

1. A surgical implant comprising:
a porous substrate including a first surface and a second surface, opposite the first surface; and
a plurality of ferromagnetic darts extending from at least one of the first and second surfaces.

2. The surgical implant of claim 1, wherein at least one of the plurality of ferromagnetic darts is made from a bioabsorbable material.

3. The surgical implant of claim 1, wherein at least one of the plurality of darts is made from a non-absorbable material.

4. The surgical implant of claim 2 or claim 3, wherein the at least one dart is formed from a ferromagnetic metal.

5. The surgical implant of claim 4, wherein the metal is iron or alloys thereof.

6. The surgical implant of claim 4, wherein the metal is selected from the group consisting of cobalt, nickel, gadolinium, dysprosium, and alloys thereof.

7. The surgical implant of any one of claim 2 to 6, wherein the material is a polymer including ferromagnetic particles.

8. The surgical implant of claim 7, wherein the ferromagnetic particles are admixed with the polymer.

9. The surgical implant of claim 7, wherein the ferromagnetic particles are coated on the polymer.

10. The surgical implant according to any preceding claim, wherein the darts each include:
a base portion fixedly associated with the porous substrate;
a body portion extending from the base portion, and
a head portion extending from the body portion.

11. The surgical implant of claim 10, wherein the head portion includes a tapered tip.

12. The surgical implant of claim 10 or claim 11, wherein the head portion of the dart includes a proximal end having a diameter that is larger than the diameter of the body portion.

13. A system for securing a surgical implant to tissue, the system comprising:
a surgical implant according to any preceding claim; and
an electromagnet configured to attract the plurality of darts.
